## Europäisches Patentamt

## European Patent Office

## Office européen des brevets

(19)

(11) Veröffentlichungsnummer: **0 065 292**
B1

(12)

# EUROPÄISCHE PATENTSCHRIFT

(45) Veröffentlichungstag der Patentschrift:
08.01.86

(51) Int. Cl.⁴: **A 61 K 9/50**, B 01 J 13/00

(21) Anmeldenummer: **82104223.1**

(22) Anmeldetag: **14.05.82**

(54) **Verfahren zur Lagerung von unilamellaren Phospholipid-Vesikeln.**

(30) Priorität: **15.05.81 US 263942**

(43) Veröffentlichungstag der Anmeldung:
**24.11.82 Patentblatt 82/47**

(45) Bekanntmachung des Hinweises auf die Patenterteilung:
**08.01.86 Patentblatt 86/2**

(84) Benannte Vertragsstaaten:
**AT BE CH DE FR GB LI NL SE**

(56) Entgegenhaltungen:
**FR - A - 2 298 318**

**CHEMICAL ABSTRACTS, Band 96, Nr. 23, 7. Juni 1982,
Seite 292, Nr. 195497e, Columbus, Ohio, USA G.J.
MORRIS: "The response of liposomes to various rates of
cooling to -196oC: Effect of phospholipidcholesterol
ratio"
FEBS Letters 16, 206-212 (1971)
Cryobiology 17, 508-515 (1980)**

(73) Patentinhaber: **Gersonde, Klaus, Prof. Dr., Preusweg 69,
D-5100 Aachen (DE)**

(72) Erfinder: **Gersonde, Klaus, Prof. Dr., Preusweg 69,
D-5100 Aachen (DE)**
Erfinder: **Weiner, Murray, Dr., 8915 Spooky Ridge Lane,
Cincinnati Ohio 45242 (US)**

(74) Vertreter: **Biermann, Wilhelm, Dr.-Ing., Morillenhang 39,
D-5100 Aachen (DE)**

## Beschreibung

Die Erfindung betrifft ein Verfahren zur Lagerung von unilamellaren Phospholipid-Vesikeln, deren Membran aus Phosphatidylcholin, Phosphatidylserin und Cholesterin in einem molaren Verhältnis von 10 bis 5:4 bis 1:10 bis 3 besteht und die eine biologisch aktive Wirksubstanz in einer wässrigen Lösung enthalten und für die Inkorporierung der Wirksubstanz in lebende Zellen durch Fusion mit den Zellen bestimmt sind.

Unilamellare Phosphlipid-Vesikel dieser Art sowie Verfahren zu ihrer Herstellung sind z.B. aus der US-A 4 192 869 bekannt. Mit Hilfe dieser bekannten Lipid-Vesikel kann man die Wirksubstanz unmittelbar in Körperzellen, insbesondere in Blutzellen einschleusen, indem man die Blutzellen mit diesen Lipid-Vesikeln inkubiert. Dabei fusioniert die Lipid-Membran der Lipid-Vesikel mit der Zellmembran der Blutzellen und der Wirkstoff wird in die Blutzellen inkorporiert. Bei diesem bekannten Verfahren werden solche Wirkstoffe in die roten Blutzellen inkorporiert, die die Sauerstoff-Affinität des Hämoglobins herabsetzen, wie Inositolhexaphosphat, 2,3-Biphosphoglycerat, Adenosintriphosphat oder ähnliche Stoffe. Die so behandelten roten Blutzellen können dann einen grösseren Anteil des gebundenen Sauerstoffs abgeben, wodurch die Sauerstoffversorgung des Gewebes verbessert wird. Die herabgesetzte Sauerstoff-Affinität des Hämoglobins in den so modifizierten Erythrozyten bleibt während der gesamten Lebenszeit der roten Blutzellen erhalten.

Lipid-Vesikel mit dem genannten Membranaufbau verlieren jedoch bei der üblichen Umgebungstemperatur nach kurzer Zeit ihre Eigenschaft, die Inkorporierung des Wirkstoffs in die Blutzellen zu vermitteln. Sie fusionieren nämlich miteinander, wodurch verhältnismässig grosse multilamellare Liposome gebildet werden, die für den Prozess der Wirkstoff-Inkorporierung in die Blutzellen unbrauchbar sind. Aus diesem Grund können die genannten Lipid-Vesikel-Suspensionen unter den üblichen Bedingungen nicht länger als 1 bis 2 Tage aufbewahrt werden. Die Herstellung dieser Lipid-Vesikel erfordert jedoch eine komplizierte apparative Ausstattung und erfahrenes Personal. Da diese Voraussetzungen nicht in allen Kliniken erfüllt sind, ist die praktische Anwendung solcher Lipid-Vesikel sowohl für therapeutische Zwecke als auch für andere Zwecke, beispielsweise für Forschungszwecke, erheblich eingeschränkt.

Es ist ferner bekannt, Liposomen mit einer innerhalb der Liposomenstruktur eingeschlossenen wässrigen Wirkstofflösung in der Weise herzustellen, dass man der Phospholipid-Dispersion den wasserlöslichen Wirkstoff zusetzt, die wässrige Dispersion bei einer Temperatur von −5°C bis −40°C einfriert, wobei der Wirkstoff in den Lipidkügelchen des Phospholipids eingeschlossen oder eingekapselt wird, und anschliessend die gefrorene Dispersion auftaut (FR-A 2 298 318).

Die in diesem Fall verwendeten Phospholipide enthalten kein Cholesterin. Die auf diese Weise hergestellten Liposome haben multilamellare Strukturen und eignen sich nicht für die unmittelbare Fusion mit den Körperzellen, sondern werden in den Magen-Darm-Trakt, in Muskeln oder in Blutgefässe eingebracht, wo sie den Wirkstoff verzögert abgeben. Die für diese Verwendung erforderliche Aktivität dieser Liposomen wird durch eine Lagerung bei Umgebungstemperatur nicht beeinträchtigt.

Es ist grundsätzlich auch bekannt, Lipid-Vesikel einer Gefrier- und Auftaubehandlung zu unterwerfen, wie sich aus CRYOBIOLOGY 1982, S. 215–218, CRYOBIOLOGY 1980, S. 508–518 und FEBS LETTERS 1971, S. 206–212 ergibt. Danach weiss man, dass die Membranen von Lipid-Vesikeln bei einer Gefrier- und Auftaubehandlung in der Regel beschädigt oder zerstört werden, und dass die Vorgänge beim Einfrieren und beim Auftauen komplex sind. Dabei spielen die Zusammensetzung der Lipid-Membran und die Abkühlgeschwindigkeit eine wesentliche Rolle. Bei niedriger Abkühlgeschwindigkeit diffundiert während des Abkühlens Wasser durch die Membran nach aussen, und die Membran wird durch ausserhalb der Lipidhülle entstehende Eiskristalle verletzt oder zerstört. Bei hoher Abkühlgeschwindigkeit findet eine erhöhte Keimbildung im Innern der Vesikel statt, die zu einer Rekristallisation beim Auftauen und einer Beschädigung der Membran von innenher führt. Bei derartigen Beschädigungen der Lipidhülle tritt die eingeschlossene Flüssigkeit aus den Vesikeln aus, so dass diese ihre Wirksamkeit aus diesem Grund verlieren. Systematische Untersuchungen über den Einfluss der Lipidzusammensetzung und der Abkühlgeschwindigkeit auf das Ausmass der Zerstörung der Lipidhülle bei Lipid-Vesikeln sind nicht bekannt.

Der Erfindung liegt die Aufgabe zugrunde, ein Verfahren zur Lagerung von unilamellaren, einen Wirkstoff enthaltenden Lipid-Vesikeln zu schaffen, das eine Konservierung solcher Lipid-Vesikel-Suspensionen auch über längere Zeiträume gestattet.

Gemäss der Erfindung wird diese Aufgabe dadurch gelöst, dass unmittelbar nach der Herstellung der Lipid-Vesikel in wässrigem Medium die Lipid-Vesikel-Suspension durch Eintauchen in eine Temperatur von −196°C bis −30°C aufweisende Kühlflüssigkeit rasch abgekühlt und bis zu ihrem Einsatz bei einer Temperatur unterhalb von −20°C, und vorzugsweise unterhalb von −30°C aufbewahrt wird.

Vorzugsweise wird als Kühlflüssigkeit ein verflüssigtes Gas verwendet, wobei sich insbesondere flüssiges Propan mit einer Temperatur von −189°C bis −141°C, und vorzugsweise von −189°C bis −170°C als besonders geeignet erwiesen hat.

Überraschenderweise bleiben die tiefgefrorenen, Wirkstoff-beladenen Lipid-Vesikel unter den erfindungsgemässen Bedingungen intakt und unfragmentiert, wenn die Lipid-Vesikel nach dem Einfrieren wieder aufgetaut werden, und behalten in vollem Umfang ihre Eigenschaft zur Fusionierung mit den Körperzellen.

Gefrorene Lipid-Vesikel-Suspensionen sind bei tiefen Temperaturen über einen langen Zeitraum stabil. Sie können daher an einem zentral gelegenen Ort produziert und im gefrorenen Zustand zu Krankenhäusern und Blutbanken transportiert werden, wo sie in Kühlboxen gelagert werden und nach dem Auftauen und Verdünnen bei Bedarf sofort eingesetzt werden können.

Es hat sich ferner gezeigt, dass die eingefrorenen Lipid-Vesikel bei Temperaturen unterhalb des Gefrierpunktes der eingekapselten Lösung von –196°C bis –20°C, und vorzugsweise bei Temperaturen unterhalb von –30°C ausserordentlich lagerungsstabil sind. Während z.B. frisch präparierte Inositolhexaphosphat (IHP) enthaltende Lipid-Vesikel ihre Effektivität zum Transport von IHP in Erythrozyten in therapeutisch notwendigen Dosen innerhalb von 2 Tagen verlieren, wenn sie bei Raumtemperatur gelagert werden, behalten tiefgefrorene Lipid-Vesikel-Suspensionen monatelang ihre Wirksamkeit. Sie behalten ihre Wirksamkeit sogar über Jahre, wenn sie in nichtoxidierender Atmosphäre aufbewahrt werden.

Das Einfrieren und Auftauen der Lipid-Vesikel macht keine speziellen Apparate erforderlich. Gemäss der Erfindung wird ein die hergestellte Lipid-Vesikel-Suspension enthaltender Beutel vorzugsweise in flüssiges Propan bei einer Temperatur zwischen –190°C und –141°C eingetaucht. Die wirksamste Methode ist die des Einfrierens der Lipid-Vesikel-Suspension in flüssigem Propan bei einer Temperatur zwischen –189°C bis –170°C, d.h. bei einer Temperatur nahe dem Gefrierpunkt des Propans (–190°C), aber weit genug entfernt vom Siedepunkt des Propans.

Als weitere geeignete, nicht-wässrige Kühlbäder kommen flüssige Gase in Frage, wie z.B. Luft, Stickstoff, Sauerstoff und Ammoniak oder Mischungen von Kohlendioxyd mit organischen Substanzen wie Alkohol, Äthanol oder Chloroform. Die Einfrierzeit in flüssigem Propan ist wesentlich kürzer als z.B. in flüssigem Stickstoff.

Wenn z.B. ein Polyäthylen-Behälter, welcher die Suspension der mit dem Wirkstoff beladenen Lipid-Vesikel enthält, wie oben beschrieben, in ein Polyäthylen-Gefäss, das flüssiges Propan bei –183°C enthält, eingetaucht wird, wobei das Flüssigpropan-enthaltende Gefäss wiederum in ein Dewar-Gefäss mit flüssigem Stickstoff eingetaucht wird, so kann man sehr schnell und ohne Zerstörung der Membran-Struktur der Lipid-Vesikel die Lipid-Vesikel-Suspension einfrieren und auf diese Weise lagerungsstabile, d.h. im Temperaturbereich von –196°C bis –20°C lagerungsfähige, Lipid-Vesikel herstellen.

Gelagerte tiefgefrorene Lipid-Vesikel-Suspensionen stehen also für den Gebrauch bereit, wenn sie vor dem Gebrauch auf Raumtemperatur aufgetaut werden.

Das erfindungsgemässe Einfrieren und Auftauen der Lipid-Vesikel-Suspension stellt somit eine erhebliche Verbesserung der bekannten Methode zur Herabsetzung der Sauerstoff-Affinität in Erythrozyten durch Modifikation des Hämoglobins mit IHP dar. Die lagerungsstabilen Liposomen können nämlich in grösseren Mengen und an einem zentralen Ort hergestellt werden, so dass letztendlich ein hoher Zeitgewinn hieraus resultiert. Ein speziell ausgestattetes und aufwendiges Laboratorium und die spezielle Produktionstechnologie müssen nicht unbedingt am Ort der Anwendung der Lipid-Vesikel vorhanden sein. Die Lipid-Vesikel können daher in Portionen und in kleinen Packungen von der Grösse, die für die Modifikation einer einzelnen Einheit von Erythrozyten notwendig sind, eingefroren und transportiert werden und im gefrorenen Zustand in den Krankenhäusern und Blutbanken gelagert werden. Die gefrorenen Lipid-Vesikel können ferner so lange gelagert werden, wie dies für die Anwendung notwendig erscheint. Nach dem Auftauen können die Lipid-Vesikel schliesslich auf die gleiche Weise eingesetzt werden wie frisch präparierte Lipid-Vesikel.

In gleicher Weise können Lipid-Vesikel-Suspensionen, die zur Einschleusung von anderen biologisch aktiven Substanzen in Körperzellen dienen sollen, nach dem erfindungsgemässen Verfahren dem Schnellgefrierprozess unterworfen und ebenfalls bei Temperaturen zwischen –196°C und –20°C gelagert werden. Diese gefrorenen Lipid-Vesikel-Suspensionen werden in derselben Weise vor dem Gebrauch aufgetaut und dann wie frisch präparierte Lipid-Vesikel-Suspensionen gleicher Zusammensetzung eingesetzt. Bei den anderen biologisch aktiven Substanzen kann es sich z.B. um Antitumorstoffe, Antibiotika, Metall-chelierende Verbindungen, Hormone und Enzyme, jeweils in wässriger Lösung, handeln.

Modifizierte Blutzellen können z.B. als zirkulierende Träger fungieren, indem sie den inkorporierten Wirkstoff nach und nach freisetzen. Die Wirkstoffe können insbesondere auch die Eigenschaften der roten Blutzelle selber modifizieren. Z.B. kann die Sauerstoffreisetzungs-Eigenschaft der Erythrozyten durch die Inkorporierung von allosterischen Effektoren, wie Inositolhexaphosphat, verändert werden.

Nachfolgend wird ein bevorzugtes Ausführungsbeispiel der Erfindung beschrieben, bei dem als Wirkstoff Inositolhexaphosphat zur Modifikation der roten Blutzellen verwendet wird.

Die Modifikation der Erythrozyten kann gemäss der hier geschilderten Erfindung prinzipiell mit anderen allosterischen Wirkstoffen, die eine grössere Affinität zum Hämoglobin haben als die physiologischen Effektoren, durchgeführt werden wie mit 2.3-Bisphosphoglycerat und Adenosintriphosphat. Andere Polyphosphate, wie z.B. Inositolpentaphosphat und Inositoltetraphosphat, dürften ebenfalls geeignet sein, beispielsweise für die Modifikation von Erythrozyten bestimmter Tierspezies. Im Falle einer bestimmten Mutation des Hämoglobins, z.B. Hb Zürich, können organische Anionen der Polycarbonsäuren diese Wirkung des allosterischen Effektors haben.

Die Lipid-Vesikel bestehen aus Mischungen von Phosphatidylcholin, Phosphatidylserin und Cholesterin in einem molaren Verhältnis von 10–5:4–1:10–3. Sie sind geeignet, als Vermittler für eine irreversible Inkorporierung der oben genannten allosterischen Effektoren in rote Blutzellen benutzt zu werden. Die Methode zur Präparation der Lipid-Vesikel und die Anwendung der Lipid-Vesikel zur Herstellung modifizierter Erythrozyten sind in der US-A 4 192 869 beschrieben.

Phosphatidylcholin, Phosphatidylserin und Cholesterol sind käuflich erhältlich. Das gleiche gilt für Inositolhexaphosphat. Die bevorzugte Lipid-Zusammensetzung umfasst Phosphatidylcholin, Phosphatidylserin und Cholesterin in einem molaren Verhältnis von 8:2:7.

Die allgemeinen Methoden zur Präparation von Lipid-Vesikeln sind bekannt. Die Lipide werden in einem organischen Lösungsmittel aufgelöst, wobei eine homogene Lösung gebildet wird. Das organische Lösungsmittel wird durch Verdampfen entfernt. Der resultierende Lipid-Film wird dann durch Schütteln mit einer wässrigen Lösung, die den biologisch aktiven Wirkstoff enthält, z.B. einen allosterischen Effektor, geschüttelt. Vorzugsweise wird die Lipid-Suspension durch Energie- und Frequenz-kontrollierte Beschallung mit Ultraschall-Energie in Lipid-Vesikel überführt, welche kleinste Tropfen der wässrigen Lösung umschliessen. Vorzugsweise wird für den angegebenen Zweck die Lipid-Mischung in einer gepufferten und nahezu mit Inositolhexaphosphat gesättigten Lösung bei pH 7–8 beschallt. Die Lipid-Konzentration kann dabei im Bereich von 17 µg/ml bis ungefähr 200 µg/ml-Lösung variieren. Nach der Ultraschall-Behandlung mit einer Energie von mehr als 100 W/cm² erhält man eine Suspension von unilamellaren Lipid-Vesikeln mit einem Durchmesser von < 500 Å, in welche die gepufferte und gesättigte wässrige IHP-Lösung eingeschlossen ist. Die so erhaltene Lipid-Vesikel-Suspension wird sodann unmittelbar im Anschluss an ihre Herstellung im Ultraschallfeld durch Eintauchen in flüssiges Propan sehr schnell abgekühlt und tiefgefroren. Wenn die Vesikel-Suspension für ihren bestimmungsgemässen Zweck eingesetzt werden soll, wird die tiefgefrorene Suspension aufgetaut. Die resultierende Suspension kann dann nach Bedarf mit Wasser, Puffer oder zusätzlicher IHP-Lösung vor der Inkubation mit Erythrozyten verdünnt werden.

Die Salz-Konzentration des Aussenmediums, in welcher die Lipid-Vesikel suspendiert sind, kann durch Verdünnung von 1/10 der Konzentration bis zu äquimolarer Konzentration des Innenmediums der Vesikel eingestellt werden. So wird z.B. eine Suspension, die 170 µg/ml Phosphatidylcholin, Phosphatidylserin und Cholesterin mit einem molaren Verhältnis von 8:2:7 enthält, eine Stunde beschallt, die resultierende Suspension der Lipid-Vesikel unmittelbar darauf eingefroren, und nach dem Auftauen unmittelbar vor der Anwendung im Verhältnis 1:1 mit Wasser verdünnt.

Vor Inkubation mit den IHP-enthaltenden Lipid-Vesikeln sind die Erythrozyten vom Blutplasma zu trennen und zu waschen. Die Erythrozyten werden dann mit der Suspension der Lipid-Vesikel gemischt und bei pH 7–8 bei einer Temperatur zwischen 18 und 37°C während 0,5–2 Stunden inkubiert; vorzugsweise wird 1 Stunde lang bei 37°C inkubiert. Die roten Blutzellen, die auf diese Weise durch Aufnahme von IHP modifiziert wurden, wobei das IHP in den roten Blutzellen an das Hämoglobin gebunden wird, werden nun sorgfältig gewaschen, um das extrazellulare IHP zu beseitigen. Die modifizierten roten Blutzellen werden in Blutplasma oder in einem bekannten Blutplasma-Ersatz resuspendiert und in den Patienten, der eine erhöhte Sauerstoffversorgung seiner Gewebe benötigt, retransfundiert. Die Erythrozyten können sowohl in das gleiche Individuum retransfundiert werden, oder aber in andere Individuen der gleichen Spezies und des gleichen Antigen-Typs. Die modifizierten roten Blutzellen des Spenders, die dem Patienten verabreicht werden, müssen vorher den entsprechenden und bekannten Blut-Charakterisierungsmethoden unterworfen werden. Das Blut oder der Blutersatz, das die modifizierten Erythrozyten enthält, kann unmittelbar in den Patienten, der dieses benötigt, transfundiert werden, oder auch unter Standardbedingungen in der Blutbank bis zu seinem Gebrauch gelagert werden.

Das folgende Beispiel illustriert, wie die Liposomen-Aktivität während Monaten der Lagerungszeit erfolgreich aufrechterhalten werden kann: Lipid-Vesikel, aufgebaut aus Phosphatidylcholin, Phosphatidylserin und Cholesterin in einem molaren Verhältnis von 8:2:7, mit einer Gesamt-Lipid-Konzentration von 170 µg/ml, mit einer 0,19 M IHP-Lösung als Einschlussmedium und suspendiert in einer 0,19 M IHP-Lösung, die mit 0,068 M bis-Tris pH 7,4 gepuffert war, wurden bei –183°C eingefroren und danach bei –30°C gelagert.

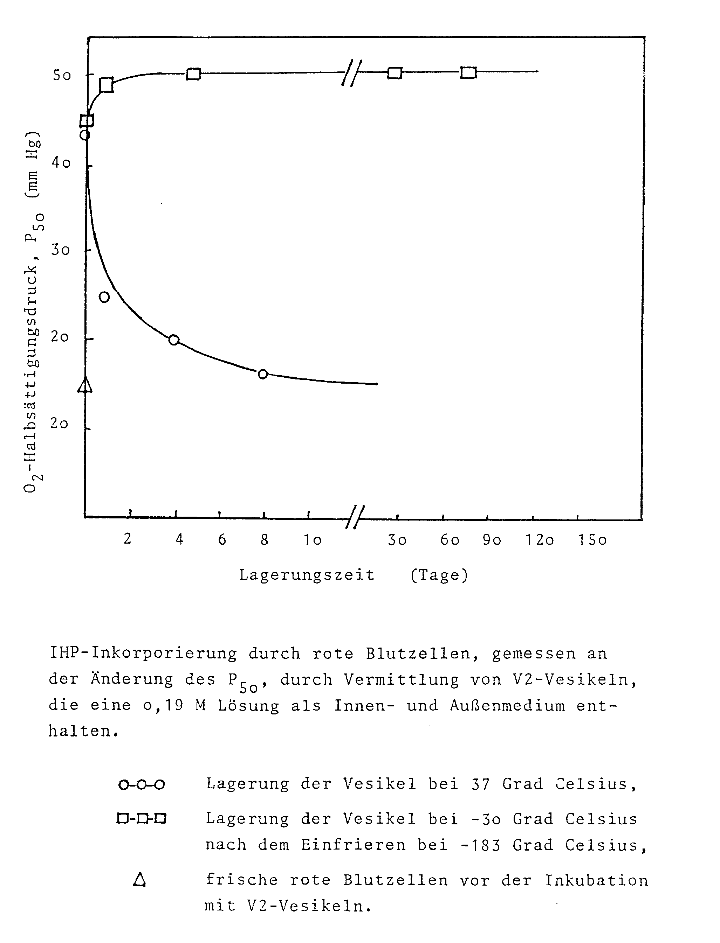

Proben dieser gefrorenen Lipid-Vesikel-Suspensionen wurden nach verschieden langen Lagerungsperioden bei Raumtemperatur aufgetaut und anschliessend mit Human-Erythrozyten inkubiert. Dann wurde der Halbsättigungsdruck, $P_{50}$, mit dem Halbsättigungsdruck von solchen roten Blutzellen verglichen, die mit Lipid-Vesikeln vorgenannter Zusammensetzung inkubiert wurden, jedoch nachdem die Vesikel bei 37°C für eine bestimmte Zeit gelagert worden waren. Frisch präparierte Lipid-Vesikel ändern den $P_{50}$ der roten Blutzellen von 15 mmHg nach 44 mmHg. Die bei 37°C gelagerten Vesikel verlieren ihre Fähigkeit zur Vermittlung der IHP-Aufnahme durch die roten Blutzellen innerhalb von 8 Tagen vollständig. Die Halb-Lebenszeit der Vesikel beträgt bei dieser Temperatur ungefähr einen Tag. Vesikel, welche dagegen bei –183°C eingefroren und bei –30°C gelagert wurden, zeichnen sich durch eine konstante Eigenschaft aus, die IHP-Aufnahme über eine Periode von 2,5 Monaten zu vermitteln. Die Abbildung zeigt, dass der $P_{50}$-Wert der roten Blutzellen, die mit Vesikeln inkubiert wurden, die bei tiefer Temperatur gelagert und nach dem Auftauen inkubiert wurden, konstant bleibt und sogar einen geringfügig erhöhten Wert aufweist.

**Patentansprüche**

1. Verfahren zur Lagerung von unilamellaren Phospholipid-Vesikeln, deren Membran aus Phosphatidylcholin, Phosphatidylserin und Cholesterin in einem molaren Verhältnis von 10 bis 5:4 bis 1:10 bis 3 besteht und die eine biologisch aktive Wirksubstanz in einer wässrigen Lösung enthalten und für die Inkorporierung der Wirksubstanz in lebende Zellen durch Fusion mit den Zellen bestimmt sind, dadurch gekennzeichnet, dass unmittelbar nach der Herstellung der Lipid-Vesikel in wässrigem Medium die Lipid-Vesikel-Suspension durch Eintauchen in eine eine Temperatur von –196°C bis –30°C aufweisende Kühlflüssigkeit rasch abgekühlt und bis zu ihrem Einsatz bei einer Temperatur unterhalb von –20°C, und vorzugsweise unterhalb von –30°C aufbewahrt wird.

2. Verfahren nach Anspruch 1, dadurch gekennzeichnet, dass als Kühlflüssigkeit ein verflüssigtes Gas verwendet wird.

3. Verfahren nach Anspruch 1 und 2, dadurch gekennzeichnet, dass als Kühlflüssigkeit flüssiges Propan mit einer Temperatur von –189°C bis –141°C, und vorzugsweise mit einer Temperatur von –189°C bis –170°C verwendet wird.

4. Verfahren nach einem oder mehreren der Ansprüche 1 bis 3, dadurch gekennzeichnet, dass das die Kühlflüssigkeit für die Gefrierbehandlung der Lipid-Vesikel-Suspension enthaltende Gefäss seinerseits in flüssigen Stickstoff eingetaucht wird.

5. Verfahren nach einem oder mehreren der Ansprüche 1 bis 4, dadurch gekennzeichnet, dass die für die Gefrierbehandlung vorgesehene Lipid-Vesikel-Suspension in einem Polyäthylen-Gefäss eingeschlossen ist.

6. Verfahren nach einem oder mehreren der Ansprüche 1 bis 5, dadurch gekennzeichnet, dass die tiefgefrorene Lipid-Vesikel-Suspension in einer nichtoxydierenden Atmosphäre aufbewahrt wird.

7. Verfahren nach einem oder mehreren der Ansprüche 1 bis 6, dadurch gekennzeichnet, dass die Membran der Phospholipid-Vesikel aus Phosphatidylcholin, Phosphatidylserin und Cholesterin in einem molaren Verhältnis von 8:2:7 besteht.

8. Verfahren nach einem oder mehreren der Ansprüche 1 bis 7, dadurch gekennzeichnet, dass die biologisch aktive Wirksubstanz Inositolhexaphosphat ist.

9. Verfahren nach einem oder mehreren der Ansprüche 1 bis 7, dadurch gekennzeichnet, dass die biologisch aktive Wirksubstanz ein Antitumorstoff, ein Antibiotikum, eine metallchelierende Verbindung, ein Hormon oder ein Enzym ist.

IHP-Inkorporierung durch rote Blutzellen, gemessen an der Änderung des $P_{50}$, durch Vermittlung von V2-Vesikeln, die eine 0,19 M Lösung als Innen- und Aussenmedium enthalten.

O-O-O Lagerung der Vesikel bei 37 Grad Celsius,
□-□-□ Lagerung der Vesikel bei –30 Grad Celsius nach dem Einfrieren bei –183 Grad Celsius,
△ frische rote Blutzellen vor der Inkubation mit V2-Vesikeln.

**Claims**

1. Process for storing of unilamellar phospholipid vesicles, the membrane of which comprising phosphatidylcholine, phosphatidylserine and cholesterol in a molar ratio of 10 to 5:4 to 1:10 to 3, in which is encapsulated a biologically active agent in an aqueous solution and which are adapted for the incorporation of the active agent in living cells by fusion with the cells, characterized in that immediately after the preparation of the lipid vesicles in an aqueous medium the lipid vesicle suspension is rapidly frozen by immersion in a cooling liquid at a temperature of –196°C to –30°C, and is stored until needed at a temperature below of –20°C, and preferentially below of –30°C.

2. Process according to claim 1, characterized in that a liquified gas is used as cooling liquid.

3. Process according to claim 1 and 2, characterized in that liquid propane at a temperature of –189°C to –141°C, and preferentially at a temperature of –189°C to –170°C is used as cooling liquid.

4. Process according to one or more of the claims 1 to 3, characterized in that the receptacle containing the cooling liquid for the freezing treatment is for his part immersed in liquid nitrogen.

5. Process according to one or more of the claims 1 to 4, characterized in that the liquid vesicle suspension provided for the freezing treatment is enclosed in a polyethylene receptacle.

6. Process according to one or more of the claims 1 to 5, characterized in that the deep-frozen lipid vesicle suspension is stored in a nonoxidizing atmosphere.

7. Process according to one or more of the claims 1 to 6, characterized in that the membrane of the phospholipid vesicles consists of phosphatidylcholine, phosphatidylserine and cholesterol in a molar ratio of 8:2:7.

8. Process according to one or more of the claims 1 to 7, characterized in that the biologically active agent includes inositol hexaphosphate.

9. Process according to one or more of the claims 1 to 7, characterized in that the biologically active agent includes an antitumor-agent, an antibiotic, a metal chelate compound, an hormone or an enzyme.

**Revendications**

1. Procédé de stockage de vésicules phospholipidiques unilamellaires dont le membrane consiste en phosphatidyl-choline, phosphatidyl-serine et cholesterine dans une proportion molaire de 10 à 5:4 à 1:10 à 3, et qui contiennent une substance biologiquement active et sont destinées à l'incorporation de la substance active dans de cellules vivantes par fusion avec les cellules, caractérisé en ce que immédiatement après la formation des vésicules lipidiques dans un milieu aqueux la suspension des vésicules lipidiques est fortement refroidie par trempe dans un liquide refroidissant ayant une température de −196 à −30°C, et est conservée, jusqu'à son usage, à une température au dessous de −20°C, et de préférence au dessous de -30°C.

2. Procédé selon revendication 1, caractérisé en ce qu'on utilise comme liquide refroidissant un gaz liquefié.

3. Procédé selon revendication 1 et 2, caractérisé en ce qu'on utilise comme liquide refroidissant propane liquide à une température de −189°C à −141°C, et de préférence à une température de −189°C à −170°C.

4. Procédé selon une ou plusieures des revendications 1 à 3, caractérisé en ce que le récipient contenant le liquide refroidissant pour le traitement frigorifiant de la suspension des vésicules lipidiques est immergé, de sa part, dans d'azot liquide.

5. Procédé selon une ou plusieures des revendications 1 à 4, caractérisé en ce que la suspension des vésicules lipidiques prévue pour le traitement frigorifiant est enfermée dans un récipient en polyethylene.

6. Procédé selon une ou plusieures des revendications 1 à 5, caractérisé en ce que la suspension frigorifiée des vésicules lipidiques est gardée dans une atmosphère non oxydante.

7. Procédé selon une ou plusieures des revendications 1 à 6, caractérisé en ce que la membrane des vésicules phospholipidiques consiste en phosphatidyl-choline, phosphatidyl-serine et cholesterine dans une proportion molaire de 8:2:7.

8. Procédé selon une ou plusieures des revendications 1 à 7, caractérisé en ce que la substance biologiquement active est inositol-hexaphosphate.

9. Procédé selon une ou plusieures des revendications 1 à 7, caractérisé en ce que la substance biologiquement active est un médicament antitumeur, un antibiotique, un composé chelaté de métal, une hormone ou un enzyme.

IHP-Inkorporierung durch rote Blutzellen, gemessen an
der Änderung des $P_{50}$, durch Vermittlung von V2-Vesikeln,
die eine o,19 M Lösung als Innen- und Außenmedium enthalten.

| | |
|---|---|
| O-O-O | Lagerung der Vesikel bei 37 Grad Celsius, |
| □-□-□ | Lagerung der Vesikel bei -3o Grad Celsius nach dem Einfrieren bei -183 Grad Celsius, |
| △ | frische rote Blutzellen vor der Inkubation mit V2-Vesikeln. |